(19) Europäisches Patentamt
European Patent Office
Office européen des brevets

(11) **EP 3 374 754 B1**

(12) **FASCICULE DE BREVET EUROPEEN**

(45) Date de publication et mention de la délivrance du brevet:
**31.08.2022 Bulletin 2022/35**

(21) Numéro de dépôt: **16791334.2**

(22) Date de dépôt: **20.10.2016**

(51) Classification Internationale des Brevets (IPC):
**G01N 17/00** *(2006.01)* **G01N 7/00** *(2006.01)*

(52) Classification Coopérative des Brevets (CPC):
**G01N 17/006; G01N 7/00;** G01N 33/2025

(86) Numéro de dépôt international:
**PCT/EP2016/075200**

(87) Numéro de publication internationale:
**WO 2017/080780 (18.05.2017 Gazette 2017/20)**

(54) **CAPTEUR POUR LA MESURE DE LA FRAGILISATION DES ACIERS PAR L'HYDROGENE DANS UN ENVIRONNEMENT AGRESSIF, LE DIT CAPTEUR COMPORTANT UNE CAVITE METALLIQUE RELIEE A UN DISPOSITIF DE MESURE DE PRESSION.**

SENSOR ZUR MESSUNG DER VERSPRÖDUNG VON STÄHLEN DURCH WASSERSTOFF IN EINER AGGRESSIVEN UMGEBUNG, WOBEI DER SENSOR EINEN METALLHOHLRAUM AUFWEIST, DER MIT EINER DRUCKMESSVORRICHTUNG VERBUNDEN IST

SENSOR FOR MEASURING THE EMBRITTLEMENT OF STEELS BY HYDROGEN IN AN AGGRESSIVE ENVIRONMENT, SAID SENSOR COMPRISING A METAL CAVITY CONNECTED TO A PRESSURE-MEASURING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **09.11.2015 FR 1560718**

(43) Date de publication de la demande:
**19.09.2018 Bulletin 2018/38**

(73) Titulaire: **IFP Energies nouvelles**
**92500 Rueil-Malmaison (FR)**

(72) Inventeurs:
• **BOUDOU, Christian**
**69780 Mions (FR)**
• **KITTEL, Jean**
**69003 Lyon (FR)**
• **PARRAIN, Gilbert**
**38790 Diémoz (FR)**

(74) Mandataire: **IFP Energies nouvelles**
**Département Propriété Industrielle**
**Rond Point de l'échangeur de Solaize**
**BP3**
**69360 Solaize (FR)**
(56) Documents cités:
**JP-A- 2011 179 893    JP-A- 2015 090 314**
**JP-B2- 2 790 702    US-A1- 2008 233 010**
**US-B1- 6 537 824**

## Description

CONTEXTE DE L'INVENTION

La présente invention décrit un capteur destiné à mesurer le risque de fragilisation par l'hydrogène (FPH) d'un métal donné dans un environnement agressif favorisant la pénétration de l'hydrogène dans le métal en question. Ce capteur est exposé à un environnement agressif susceptible de provoquer une fragilisation par l'hydrogène pour le métal constitutif du corps métallique. La pénétration dans le métal d'hydrogène depuis le milieu extérieur conduit à la diffusion d'hydrogène dans le métal du capteur, puis à sa recombinaison en hydrogène gazeux à l'intérieur de la cavité fermée.

La mesure de pression stationnaire atteinte dans cette cavité est alors indicative de l'activité de l'hydrogène dans l'acier. Si la teneur limite en hydrogène admissible dans le métal considéré est connue, le capteur selon l'invention permet alors la vérification en temps réel que ce niveau n'est pas atteint en service.

Le capteur selon l'invention s'applique plus particulièrement à la mesure du risque de fragilisation d'aciers utilisés dans différents équipements industriels. Mais il pourrait être utilisé également dans la mesure du risque de fragilisation d'autres matériaux métalliques. Dans la suite du texte on parlera le plus souvent d'acier, sans que cela constitue une limitation à ce métal particulier.

La fragilisation par l'hydrogène est un phénomène relativement fréquent dans les avaries d'équipements industriels en matériaux métalliques, avec des conséquences parfois désastreuses. L'origine physique de ce phénomène vient de la facilité de diffusion de l'hydrogène dans la plupart des métaux, du fait de sa très petite taille (c'est le plus petit des atomes). Lorsque les environnements agressifs auxquels sont soumis les métaux contiennent de l'hydrogène, celui-ci peut alors éventuellement pénétrer dans l'acier.

Une grande diversité d'environnement est concernée, par exemple : milieu gazeux contenant de l'hydrogène, milieux aqueux corrosifs dans lesquels une réaction de réduction mettant en œuvre un composé hydrogéné (comme l'eau ou comme les ions H+ en milieu acide) se produit.

Une fois dans l'acier, l'hydrogène peut donc diffuser assez facilement, et éventuellement s'accumuler dans des zones métallurgiques favorables, comme des défauts cristallins (dislocations, lacunes, précipités), des joints de grains, des inclusions.

Cette accumulation d'hydrogène conduit à un affaiblissement des propriétés mécaniques du métal.

Si le métal est soumis à des contraintes (externes ou résiduelles), et que la résistance mécanique associée à l'hydrogène diminue en-dessous des contraintes appliquées, une fissuration locale peut se produire. Les contraintes en question peuvent avoir différentes sources : contraintes résiduelles locales associées aux défauts métallographiques, contraintes issues des étapes de mise en forme, contraintes de service des équipements (poids, pression interne...). Ensuite, avec l'accumulation d'hydrogène gazeux sous haute pression dans les cavités formées, une propagation de la fissuration peut se produire.

Ce type de fissuration représente un enjeu industriel important, dans la mesure où il s'agit d'un phénomène généralement soudain, sans signes précurseurs évidents, et pouvant conduire à des ruptures complètes d'équipement. Il existe donc un réel intérêt à disposer d'un capteur permettant d'anticiper la survenue de ce risque de ruptures

DESCRIPTION SOMMAIRE DES FIGURES

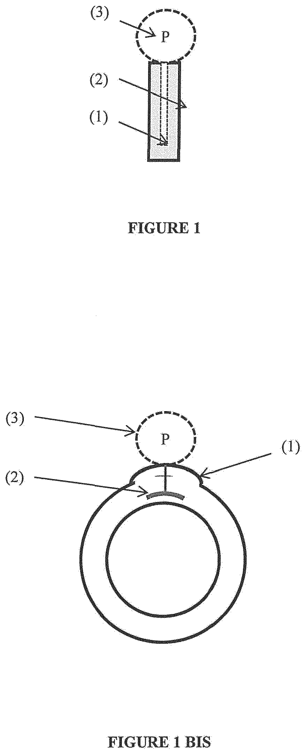

La figure 1 représente une vue schématique du capteur illustratif qui ne fait pas partie de l'invention, celui-ci ayant une forme tubulaire et étant relié à un capteur de pression. La forme tubulaire est pratique, mais d'autres formes pourrait être utilisées, sphérique ou plane par exemple.

La figure1 bis représente une vue schématique du capteur selon l'invention lorsque celui-ci est directement intégré à l'équipement industriel à caractériser. Dans ce cas le capteur selon l'invention est ménagé dans une portion de l'équipement à caractériser, cette portion étant si besoin munie d'une surépaisseur. Les figures suivantes servent à illustrer les exemples fournis en fin de texte.

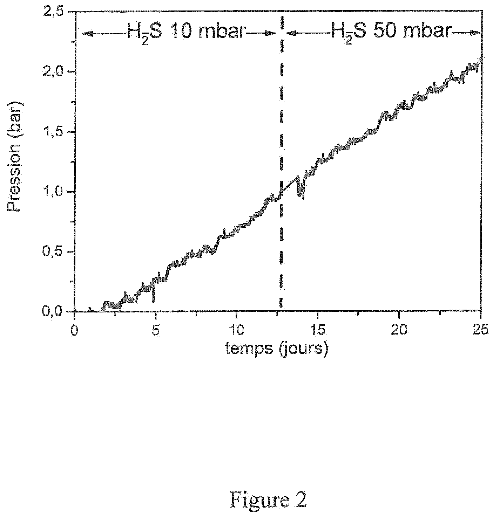

La Figure 2 représente l'évolution dans le temps de la pression interne dans un capteur creux en acier faiblement allié à haute limite d'élasticité selon l'invention, ce capteur étant soumis à un environnement corrosif constitué d'eau à 35 g/L NaCl à pH 4,5 et contenant 10 puis 50 mbar d'H2S dissous. Ce type d'environnement est connu pour favoriser l'entrée d'hydrogène dans l'acier. Il provient dans ce cas de la réaction électrochimique de réduction du proton ($H^+ + e^- \rightarrow H$)

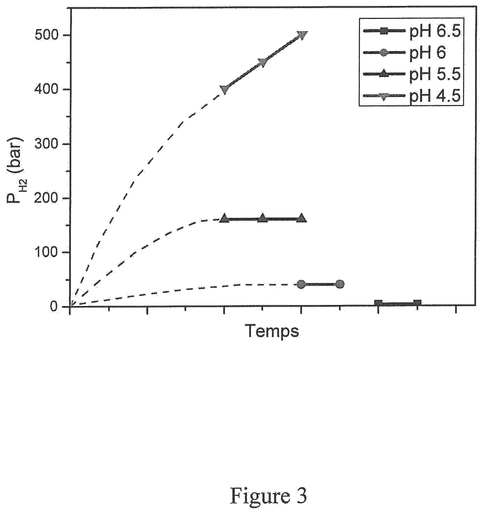

La figure 3 représente des mesures de pression d'équilibre dans le capteur creux selon l'invention en acier faiblement allié ferrito-perlitique exposé à des solutions corrosives à différents pH et sous 100 mbar d'$H_2S$. Ce type d'environnement est connu pour favoriser l'entrée d'hydrogène dans l'acier. Il provient dans ce cas de la réaction électrochimique de réduction du proton ($H^+ + e^- \rightarrow H$)

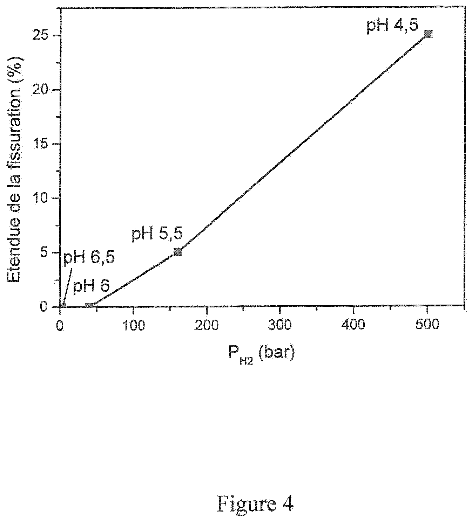

La figure 4 représente le lien entre étendue de fissuration et pression d'équilibre pour un acier faiblement allié ferrito-perlitique exposé à une solution d'eau à différents niveaux de pH et sous 100 mbar d'$H_2S$.

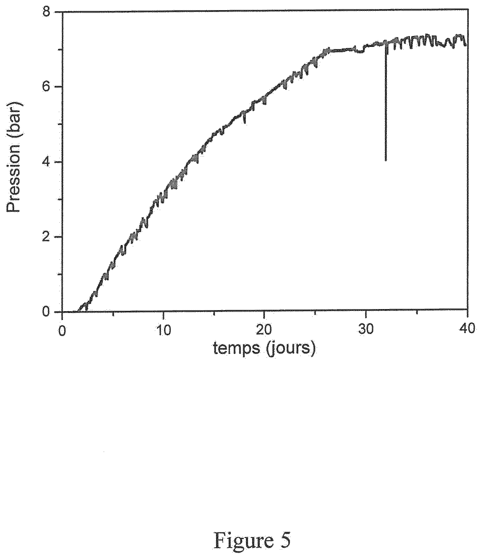

La figure 5 représente l'évolution de la pression interne dans un capteur creux en acier faiblement allié ferrito-perlitique selon l'invention, ce capteur étant soumis à un environnement corrosif constitué d'eau à 35 g/L NaCl à pH 6 et contenant 10 mbar d'H$_2$S dissous. Ce type d'environnement est connu pour favoriser l'entrée d'hydrogène dans l'acier.

Il provient dans ce cas de la réaction électrochimique de réduction du proton (H$^+$ + e$^-$ → H)

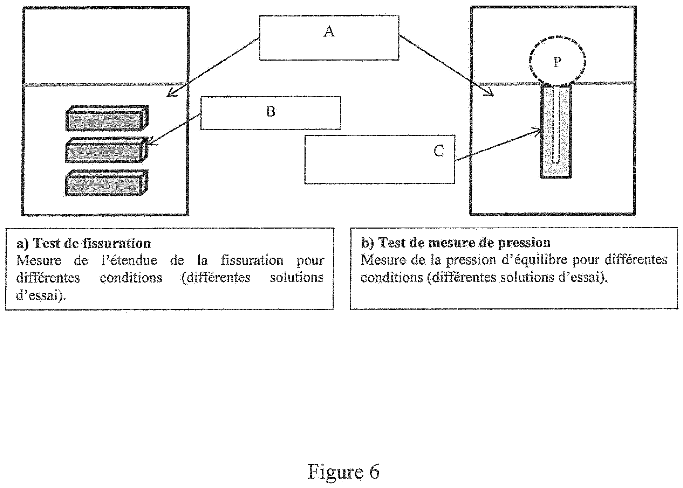

La figure 6 représente sous forme schématique la méthodologie suivie pour réaliser l'exemple 2 avec une première partie de l'expérience utilisant des coupons de l'acier à étudier (B), et une seconde partie de l'expérience utilisant le capteur selon l'invention (C), les deux étant immergés dans un milieu agressif contenant de l'hydrogène (A).

## EXAMEN DE L'ART ANTERIEUR

Afin d'évaluer les risques de fragilisation par l'hydrogène sur des équipements industriels, deux grandes familles de méthodes sont majoritairement utilisées :

- l'inspection périodique par des outils de contrôle non destructif,
- l'utilisation de capteurs visant à évaluer des flux d'hydrogène traversant le métal.

L'inspection périodique a pour but principal de détecter la présence d'éventuelles fissures, avec un seuil de détection aussi fin que possible, et/ou de suivre l'évolution dans le temps de fissures déjà détectées lors d'une précédente inspection.

Ce type de méthode présente toutefois un niveau de risque assez élevé, lié d'une part au caractère généralement rapide de la propagation des fissures après amorçage, et d'autre part au caractère localisé des fissures, avec comme conséquence une probabilité élevée de ne pas détecter lesdites fissures par une inspection qui couvre rarement l'intégralité des équipements.

Par conséquent, l'inspection spécifique à la FPH est souvent restreinte aux équipements pour lesquels les conséquences de fissures ne sont pas trop graves , par exemple des équipements sous pression modérée avec des fluides peu dangereux.

L'utilisation de capteurs spécifiques permet un suivi plus régulier. En matière de fragilisation par l'hydrogène, le paramètre le plus souvent utilisé est le flux d'hydrogène traversant une membrane métallique.

En effet, comme indiqué en introduction, la fragilisation par l'hydrogène des métaux provient de la pénétration de l'hydrogène depuis le milieu agressif vers l'intérieur de l'acier. Ce flux d'entrée s'avère être relativement aisé à mesurer avec des dispositifs de perméation à travers une membrane, en application directe des lois de diffusion de Fick.

Les capteurs utilisés consistent en général en une membrane en acier, dont l'une des faces est exposée au milieu hydrogénant, alors que l'autre face est maintenue dans des conditions permettant à l'hydrogène de ressortir avec un dispositif de mesure de ce flux sortant.

Dans le cas d'école d'une diffusion d'hydrogène sans interaction avec le métal (diffusion purement interstitielle), la mesure du flux stationnaire (Js) permet d'estimer la concentration en hydrogène dans le métal au niveau de la face d'entrée (C0) à partir de la relation :

$$J_s = D \frac{c_0}{l} \quad \text{(équation 1)}$$

équation (1) dans laquelle D est le coefficient de diffusion de l'hydrogène dans le métal considéré, et *l* est l'épaisseur de membrane à traverser.

Plusieurs types de dispositifs de mesure du flux d'hydrogène sortant de la membrane sont utilisés.

Les plus fréquemment recensés dans la littérature scientifique sont des dispositifs électrochimiques, pour lesquels la face métallique de sortie de l'hydrogène est mise en contact avec une solution électrolytique et maintenue à un potentiel auquel l'oxydation des atomes d'hydrogène se produit spontanément et génère un courant électrique qui peut être mesuré par un dispositif de type ampèremètre.

Les principes de cette mesure électrochimique ont été introduits dans une publication de 1964 par Devanathan et Stachurski [1]. Ce type de dispositif est peu adapté à des applications de suivi d'équipements en service, en raison de la complexité de mise en œuvre, qui nécessite l'emploi d'une chambre de mesure remplie d'une solution électrolytique et équipée d'un système de mesures électrochimiques.

Quelques brevets proposent toutefois des méthodes de suivi de la fragilisation par l'hydrogène mettant en œuvre ces principes de mesure électrochimique, comme par exemple les brevets JP2011179893A, JP2013044712A, JP2013044716A.

Des dispositifs mettent en œuvre une mesure de pression. Dans ce cas, la face de sortie de l'hydrogène débouche dans une enceinte étanche dans laquelle la pression est mesurée, et le flux d'hydrogène peut alors être déduit de la vitesse de montée en pression. Ce type de capteur est généralement équipé d'un système de purge afin d'évacuer régulièrement l'hydrogène accumulé dans le capteur, et de maintenir un gradient maximum d'hydrogène au sein de la membrane.

Ce principe est par exemple appliqué dans le brevet US5279169A ou dans le brevet US6537824B.

Des dispositifs mettant en œuvre une mesure de volume de l'hydrogène dans une cavité fermée et partiellement remplie d'un liquide (brevet US4416996A).

L'évolution du volume donne ainsi une mesure directe du flux d'hydrogène sortant de la membrane, et ce flux peut alors être utilisé afin d'estimer les risques de fragilisation par l'hydrogène.

L'application d'une mesure de flux d'hydrogène afin d'évaluer les risques de fragilisation par l'hydrogène de pièces mécaniques soumises à des sollicitations mécaniques cycliques est également mentionnée dans le brevet WO13012364A1, sans que la méthode de mesure de flux d'hydrogène ne soit spécifiée.

L'évaluation de la vitesse de corrosion de la paroi interne de métal est une autre application fréquemment citée pour les dispositifs précédemment évoqués qui mesurent un flux d'hydrogène à travers une paroi métallique. Le principe de ces mesures repose sur le lien entre la quantité d'hydrogène qui entre dans l'acier et la vitesse de corrosion.

Ce lien est relativement direct, par exemple dans le cas de la corrosion en milieu aqueux acide, où la réaction cathodique est la réduction du proton donnant un atome d'hydrogène qui peut alors entrer dans le métal et diffuser. De telles méthodes pour le suivi de corrosion sont ainsi mentionnées dans les brevets US6058765A, US2013236975A.

Certaines limites peuvent être identifiées pour les dispositifs de l'art antérieur précédemment cités.

- En premier lieu, la grandeur mesurée est toujours un flux d'hydrogène à travers une paroi métallique. Une corrélation est ensuite proposée entre la valeur de ce flux et le risque de fragilisation par l'hydrogène ou la vitesse de corrosion de la paroi interne. Or, ce lien est loin d'être direct.

Il est en effet connu de l'homme de l'art que la fragilisation par l'hydrogène conduisant à la fissuration interne (phénomène de « blistering », ou « hydrogen induced cracking », noté HIC dans la terminologie anglo saxonne) est grandement liée à la quantité d'hydrogène absorbée dans le métal, et à son activité chimique dans le métal.

L'amorçage de la fissuration nécessite d'atteindre une concentration suffisante en hydrogène absorbé. Si la valeur du flux d'hydrogène est un des paramètres les plus facilement mesurables, il n'en est pas pour autant le plus pertinent : en effet, celui-ci indique la vitesse à laquelle l'hydrogène entre dans le métal, mais n'indique en rien la valeur limite (concentration ou activité en hydrogène absorbé) qui sera atteinte à l'état stationnaire. Cette valeur d'activité ou concentration d'hydrogène à l'état stationnaire est désignée Ce. Elle correspond à une pression interne d'hydrogène (par application de la loi de Sieverts), qui est désignée dans la suite du texte comme pression à l'équilibre, ou Pe. Or c'est bien le dépassement d'une concentration interne en hydrogène supérieure à un seuil donné (désigné comme concentration seuil Cs ou pression seuil Ps, selon qu'on utilise les grandeurs de concentration ou d'activité, ou les grandeurs de pression, qui sont liées entre-elles par la loi de Sieverts) qui conditionne la fissuration ou l'absence de fissuration.

Cette valeur de concentration d'équilibre (Ce) peut être estimée à l'aide des mesures de flux, mais de façon assez approximative, et en faisant de nombreuses hypothèses simplificatrices sur le mode de diffusion, le coefficient de diffusion, et l'épaisseur de paroi, en considérant que la concentration d'équilibre (Ce) est égale à la concentration en hydrogène absorbé dans le métal au niveau de la face d'entrée (C0) calculée à partir des mesures de flux et en utilisant l'équation (1).

DESCRIPTION SOMMAIRE DE L'INVENTION

La présente invention peut se définir comme un dispositif permettant la mesure du risque de fragilisation par l'hydrogène de métaux utilisés dans différents types d'installations industrielles telles que des canalisations pour le transport de brut ou de produits hydrocarbonés, ou des réacteurs chimiques tels que des réacteurs d'hydrotraitement qui peuvent travailler jusqu'à des pressions de plusieurs centaines de bars.

Plus précisément l'équipement industriel selon la présente invention peut se définir comme un équipement industriel comprenant un capteur pour la mesure du risque de fragilisation par l'hydrogène dudit équipement industriel métallique (appelé simplement métal dans la suite du texte) comprenant les éléments suivants :

- un corps métallique, dans lequel une cavité fermée est réalisée, cette cavité présentant à une de ses extrémités une communication avec un dispositif de mesure de pression,
- un dispositif de mesure de la pression relié à la cavité et permettant de mesurer la pression à l'intérieur de ladite cavité,

   ladite cavité ayant une épaisseur de paroi, mesurée entre la surface extérieure et intérieure de la cavité, comprise entre 1/3 et 1/50, et de manière préférée entre 1/4 et 1/10 de l'épaisseur de l'équipement industriel à évaluer,
   ledit capteur étant réalisé dans une partie de paroi de l'équipement industriel à évaluer.

De manière générale, le corps métallique creux constituant le capteur de fragilisation selon l'invention doit présenter des caractéristiques géométriques offrant une grande surface de métal exposé au milieu extérieur, une cavité de volume réduit afin de permettre une augmentation rapide de la pression pour un flux d'hydrogène entrant donné, et une épaisseur de paroi aussi faible que possible afin de permettre un flux aussi élevé que possible.

L'épaisseur de paroi est définie comme l'épaisseur mesurée entre la surface extérieure et l'intérieur de la cavité.

Pour un capteur selon la figure 1, le rapport volume de la cavité sur surface exposée au milieu agressif hydrogénant doit de préférence être compris entre 0,01 et 0,5 cm.

Le capteur pour la mesure du risque de fragilisation par l'hydrogène d'un métal est réalisé directement dans une partie de l'équipement industriel à évaluer, plus précisément dans une partie de paroi de réacteur ou dans une partie de paroi de canalisation.

La présente invention concerne également une méthode d'évaluation du risque de fragilisation par l'hydrogène d'un métal dans un environnement donné, utilisant le capteur selon ladite invention, dans laquelle on procède en suivant les étapes suivantes :

- Etape 1 : on procède pour un métal donné à des tests de fissuration dans des environnements hydrogénant présentant des risques de fragilisation par l'hydrogène croissants, afin d'obtenir d'une part les conditions pour lesquelles il n'y a pas de fissuration et d'autre part les conditions pour lesquelles une fissuration est rencontrée,
- Etape 2 : on procède, dans les mêmes environnements hydrogénant que pour l'étape 1, à des tests de mesure de pression à l'aide d'un capteur selon l'invention, réalisé dans le même métal que celui utilisé pour l'étape 1, et on mesure la pression d'équilibre (Pe) en H2 pour chaque environnement,
- Etape 3 : on compare pour chaque environnement les résultats des tests de fissuration et des tests de mesure de pression, et on détermine quelle est la pression minimale correspondant à des situations de fissuration. Cette pression minimale est considérée comme la valeur seuil de résistance à la fissuration pour cet acier, et est désignée Ps
- Etape 4 : on expose le capteur de fragilisation réalisé dans le même métal à un environnement donné, et on suit l'évolution dans le temps de la pression dans le capteur jusqu'à ce que celle-ci atteigne un plateau où elle devient constante. On désigne cette pression d'équilibre comme Pe.
- Etape 5 : on compare alors la valeur à l'équilibre Pe avec la valeur seuil Ps obtenue à l'étape 3 :

  *Si Pe est inférieure à Ps, il n'y a pas de risque de fissuration de l'acier dans les conditions considérées

  *si Pe est supérieure à Ps, il y a un risque de fissuration.

Le capteur selon la présente invention peut être utilisé pour évaluer le risque de fragilisation d'aciers soumis à des environnements gazeux contenant de l'H2 ou de l'H2S, ou à des environnements liquides contenant de l'H2S dissous et présentant des pH de 3 à 8, et préférentiellement de 4 à 7.

A titre purement illustratif, le capteur selon l'invention peut être utilisé dans un environnement agressif de même nature que celui de l'installation industrielle à surveiller, par exemple un milieu aqueux contenant de l'H2S dissous tel que rencontré en production pétrolière,

Toujours à titre illustratif et non limitatif, le capteur selon l'invention peut être utilisé dans un environnement agressif de même nature que celui de l'installation industrielle à surveiller, par exemple un milieu gazeux à haute température contenant de l'hydrogène tel que rencontré dans les procédés de raffinage.

DESCRIPTION DETAILLEE DE L'INVENTION

La présente invention décrit un capteur et le mode d'utilisation de ce capteur, visant à évaluer le risque de fragilisation par l'hydrogène pour un métal donné dans un environnement agressif favorisant la pénétration d'hydrogène dans le métal.

Le capteur selon l'invention est constitué d'un corps métallique comportant une cavité fermée reliée à un dispositif de mesure de pression. Ce capteur est destiné à être exposé à un environnement agressif susceptible de provoquer une fragilisation par l'hydrogène pour le métal constitutif du corps métallique.

La pénétration d'hydrogène depuis le milieu extérieur conduit à la diffusion d'hydrogène dans le métal du capteur, puis à sa recombinaison en hydrogène gazeux à l'intérieur de la cavité fermée.

La mesure de pression stationnaire (Pe) atteinte dans cette cavité est alors indicative de l'activité de l'hydrogène dans l'acier. Si la teneur seuil en hydrogène admissible dans le métal considéré est connue (Ps), le capteur permet alors la vérification en temps réel que ce niveau n'est pas atteint en service.

La présente invention décrit donc un capteur de fragilisation dont les constituants principaux sont schématisés à la figure 1 ou à la figure 1 bis selon la version utilisée.

Ce capteur comporte :

(1) une portion de métal, choisie dans la même nuance de métal que l'installation pour laquelle on souhaite évaluer le risque de fragilisation par l'hydrogène. En utilisation normale, cette portion de métal est exposée au milieu corrosif hydrogénant.
(2) une cavité de faible volume, ménagée à l'intérieur de la portion de métal (1),
(3) un dispositif de mesure de la pression à l'intérieur de la cavité.

L'approche proposée dans la présente invention est très différente dans la nature des données utilisées et dans l'interprétation desdites données par rapport à l'art antérieur.

En effet, alors que la pratique courante actuelle a pour finalité la détermination des flux d'hydrogène, nous proposons d'utiliser directement la mesure de pression à l'équilibre dans la cavité pour évaluer l'activité de l'hydrogène absorbé dans l'acier.

En effet, en application de la loi de Sieverts, l'activité d'un élément gazeux dissous dans un métal est directement proportionnelle à la racine carrée de la pression de ce même gaz en équilibre avec le métal, correspondant donc à la pression à l'équilibre (Pe) engendrée par ce gaz dans la cavité de mesure. Par conséquent, la mesure de la pression à l'équilibre à l'intérieur de la cavité du capteur peut être directement corrélée à l'activité ou concentration de l'hydrogène dans l'acier à l'équilibre (Ce). Or le risque de fissuration interne de type « blistering » ou « hydrogen induced cracking, noté en abrégé HIC » est directement relié à l'activité de l'hydrogène dans l'acier. Cette mesure de pression correspond donc à une mesure directe de l'intensité du risque de fragilisation par l'hydrogène.

Un autre élément différenciant de cette invention repose dans l'utilisation pour le corps du capteur du même métal que celui de l'équipement industriel à surveiller. En effet, pour les mesures de flux d'hydrogène selon l'art antérieur, la nature du métal constitutif de la membrane d'acier peut être choisie indifféremment dans une nuance métallique proches du métal de l'équipement à surveiller, mais pas forcément identique à celui-ci. Or, la nature du métal peut jouer sur les propriétés de diffusion et de solubilité de l'hydrogène, et surtout sur la teneur limite admissible avant que la fissuration ne se produise.

Les valeurs de concentration seuil (Cs) ou pression seuil (Ps) qui définissent à partir de quelle quantité d'hydrogène absorbé le métal est susceptible de fissurer, sont en effet propres à chaque métal ou chaque grade d'acier. Il en est de même pour les concentrations et pression stationnaires (Ce et Pe).

Il est donc important d'utiliser pour le dispositif un métal représentatif, et de manière préférée le même métal que celui qui sera utilisé pour l'équipement industriel.

L'utilisation du capteur selon l'invention repose alors sur la connaissance préalable du domaine de résistance à la fragilisation par l'hydrogène du métal considéré, lequel peut être déterminé par n'importe quelle méthode d'essai de fragilisation par l'hydrogène bien connu de l'homme de l'art.

Parmi ces méthodes on peut citer par exemple l'essai décrit dans le document NACE TM0284 (NACE International) qui décrit la réalisation d'essais de tenue à la fissuration HIC d'aciers faiblement alliés en milieu aqueux contenant de l'H2S dissous.

Cette valeur seuil de pression (Ps) peut être caractérisée à l'aide d'un dispositif capteur selon l'invention, comme illustré dans l'exemple 2. Une fois connue la valeur de seuil d'activité ou de pression d'hydrogène (Cs ou Ps) pour un métal donné, le dispositif capteur selon l'invention peut être utilisé pour s'assurer que cette valeur limite n'est pas dépassée en service.

Afin d'anticiper les risques, il est important que les épaisseurs des parois du capteur soient inférieures aux épaisseurs de métal de l'installation à surveiller, et que le volume de la cavité soit aussi faible que possible. Dans de telles conditions, le temps mis pour atteindre la pression d'équilibre (Pe) dans le capteur est plus rapide que le temps mis pour atteindre le même niveau de pression dans l'installation réelle, permettant ainsi d'anticiper les risques.

EXEMPLES SELON L'INVENTION

**Exemple 1 selon l'art antérieur : acier faiblement allié à haute limite d'élasticité**

Dans cet exemple, le corps du capteur a été réalisé à partir d'acier faiblement allié présentant une haute limite d'élasticité. Sa microstructure est ferrito-perlitique. Ce type d'acier est très sensible à la fissuration interne par l'hydrogène lorsqu'il est utilisé en présence d'eau contenant de l'$H_2S$ dissous.

Ces risques dépendent principalement du pH de la solution et de la teneur en $H_2S$.

Deux tests de résistance à la fissuration (HIC abréviation de la terminologie anglo saxonne « hydrogen induced cracking ») ont été menés sur cet acier dans une solution aqueuse à 35 g/L NaCl, à pH 4,5 et sous une pression partielle d'$H_2S$ de 10 mbar (essai 1) et 50 mbar (essai 2). Ces tests ont été menés selon la méthode NACE TM 0284 bien connue de l'homme de l'art, et avec des durées d'immersion de 1 mois. Ils ont montré pour l'essai 2 (50 mbar d'$H_2S$) une fissuration importante de l'acier, alors que pour l'essai 1 (10 mbar d'$H_2S$), aucune fissuration n'a été constatée.

Des tests selon l'art antérieur, mettant en œuvre un capteur de pression utilisé pour déterminer un flux d'hydrogène à travers l'acier, ont ensuite été menés dans une solution aqueuse de même composition que pour les précédents essais (35 g/L NaCl, à pH 4,5) et en faisant varier en cours d'essai la composition en $H_2S$ de 10 à 50 mbar. Ces conditions d'essai correspondent donc au démarrage de l'essai à un environnement où le matériau n'est pas sensible à la fissuration interne (pH 4,5 et 10 mbar d'H2S), puis à un environnement où le matériau est sensible à la fissuration (pH 4,5 et 50 mbar d'$H_2S$).

La courbe d'évolution de pression interne mesurée tout au long de cet essai est présentée en Figure 2.

Cette courbe révèle que le changement de teneur en H2S de 10 à 50 mbar ne se traduit pas par un changement de la vitesse de montée en pression. Cette vitesse de montée en pression est une traduction directe du flux d'hydrogène traversant la paroi métallique. C'est cette valeur de flux qui est citée dans l'art antérieur pour caractériser le risque de fragilisation par l'hydrogène. Cet exemple montre ainsi que l'utilisation d'un simple capteur de mesure de flux tel que décrit dans l'état de l'art n'est pas capable de détecter une différence entre ces deux environnements corrosifs, alors même que le premier sous 10 mbar d'$H_2S$ ne présente pas de risque de fissuration pour cet acier, et que le second sous 50 mbar d'$H_2S$, conduit au contraire à une fissuration importante de cet acier.

Cet illustre les limites des pratiques actuelles décrites dans l'art antérieur, consistant uniquement en des mesures de flux. Le second exemple vise à illustrer de manière plus directe l'intérêt des mesures de pression d'équilibre par utilisation du capteur selon l'invention.

**Exemple 2** illustratif ne formant pas partie de l'invention :
**acier faiblement allié pour tôle de canalisation de type API 5L X65**

Dans cet exemple, l'acier testé est un acier faiblement allié de type API 5L X65, couramment utilisé pour la fabrication de canalisations de transport de pétrole et de gaz. Cet acier présente des risques de fragilisation par l'hydrogène lorsqu'il est utilisé en présence d'eau contenant de l'$H_2S$ dissous.

Ces risques dépendent principalement du pH de la solution et de la teneur en $H_2S$.

Des tests de résistance à la fissuration (noté en abrégé « HIC », abréviation de la terminologie anglo saxonne « hydrogen induced cracking ») ont été menés sur cet acier dans des solutions de pH variant entre 4,5 et 6,5, sous une pression partielle de 100 mbar en $H_2S$.

Ces tests ont été menés sur des coupons en acier de 100 mm de long, 20 mm de large, et d'épaisseur équivalente à l'épaisseur de tôle (soit 17 mm) selon la méthode NACE TM 0284 bien connue de l'homme de l'art, et avec des durées d'immersion de 1 mois.

Ces tests selon l'art antérieur, permettent de vérifier, pour un environnement corrosif donné, si l'acier présente des risques de fissuration interne. En cas de fissuration, celle-ci peut-être quantifiée par exemple par contrôle non destructif par ultrasons. L'étendue de la fissuration est alors exprimée en pourcentage de surface fissurée selon un plan donné, et désignée par le sigle CAR (abréviation de « Crack Area Ratio » dans la terminologie anglo saxonne).

Le critère CAR, bien connu de l'homme de l'art, permet de quantifier l'étendue de la fissuration interne, qui peut varier entre 0 % pour une absence de fissuration, à 100 % pour un échantillon totalement fissuré. Ces tests sont désignés dans la suite de l'exemple comme « tests de fissuration ».

Pour les mêmes conditions d'essai (même pH et même pression partielle d'$H_2S$), des tests selon l'invention ont été menés à l'aide d'un capteur creux réalisé avec le même acier, afin de déterminer la pression d'équilibre (Pe) en hydrogène atteinte dans les conditions de l'essai. Ces tests sont désignés dans la suite de l'exemple comme « tests de mesure de pression ».

La figure 6 illustre les deux types d'essais réalisés.

Les résultats de l'étendue de fissuration pour cet acier, obtenus à partir des test de fissuration dans les différentes conditions d'essai sont donnés dans le Tableau 1 ci-dessous. Comme attendu, la sévérité de l'environnement d'essai varie fortement avec le pH de la solution, avec un seuil à pH 6 au-dessus duquel il n'y a plus de fissure détectable.

TABLEAU 1 : Corrélation entre le pH de l'environnement de test et le CAR (mesuré par des tests de fissuration) ainsi que la pression d'équilibre d'H2 (Pe) (mesurée par des tests de mesure de pression)

| pH | 4,5 | 5,5 | 6 | 6,5 |
|---|---|---|---|---|
| CAR (%) | 25 | 5 | 0 | 0 |
| Pe (bar) | > 500 | 160 | 40 | 3 |

Pour cet acier, et dans un milieu d'essai contenant 100 mbar d'$H_2S$, une série de tests de mesure de pression a été menée à l'aide d'un dispositif capteur creux selon l'invention, afin de déterminer les pressions d'équilibre (Pe) correspondant aux différents niveaux de pH. Afin d'éviter la fissuration des corps en acier lors des test de mesure de pression, le prélèvement a été réalisé dans une zone d'acier éloignée du centre de la tôle, qui est la plus sensible vis-à-vis de la fragilisation par l'hydrogène en raison d'une concentration plus élevée en inclusions.

Malgré ces précautions, certains tests de mesure de pression réalisés à pH 4,5 ont dû être interrompu en raison de fuites associées à des fissures dans le corps du capteur.

Le tableau 1 corrèle donc l'étendue de la fissuration (CAR) avec la pression d'équilibre (Pe) pour cet acier faiblement allié de microstructure ferrito-perlitique exposé à des milieux aqueux contenant 35 g/L de NaCL sous 100 mbar d'$H_2S$ et à différents pH.

Les résultats sont illustrés par les figures 3 et 4.

En comparant ces mesures de pression d'équilibre (tests de mesure de pression) avec les mesures de l'étendue de fissuration (tests de fissuration), il est possible de conclure que pour cet acier, la limite de résistance à la fissuration correspond à une pression seuil en hydrogène Ps de 40 bar comme indiqué sur la figure 4. La figure 4 établit le lien existant pour un acier donné entre l'étendue de la fissuration et la pression en H2 dans le capteur de fragilisation.

Un capteur de fragilisation construit dans cette nuance d'acier peut donc maintenant être utilisé dans n'importe quel environnement hydrogénant, afin de vérifier que la pression seuil (Ps) de 40 bar n'est pas dépassée.

Ce principe a donc été utilisé pour un essai à 10 mbar d'$H_2S$ et à pH 6.

La courbe d'évolution de pression est présentée en figure 5.

Il apparaît très clairement que la pression d'équilibre (Pe) s'établit dans ces conditions à 7 bar, bien inférieure au seuil de 40 bar établi pour ce matériau. Dans ce cas, l'utilisation du capteur selon l'invention conduit à prédire une absence de risque de fissuration.

Des tests de fissuration menés dans ces mêmes conditions ont confirmé l'absence de fissuration, comme prévu par les mesures réalisées à l'aide du capteur de fragilisation.

## Revendications

1. Equipement industriel, ledit équipement industriel étant un réacteur ou une canalisation, comprenant un capteur pour la mesure du risque de fragilisation par l'hydrogène du métal de l'équipement industriel à évaluer, comprenant les éléments suivants :

   - un corps métallique, dans lequel une cavité fermée est réalisée, cette cavité présentant à une de ses extrémités une communication avec un dispositif de mesure de pression,
   - un dispositif de mesure de la pression relié à la cavité et permettant de mesurer la pression à l'intérieur de ladite cavité,
   - ladite cavité ayant une épaisseur de paroi, mesurée entre la surface extérieure et intérieure de la cavité, comprise entre 1/3 et 1/50, et de manière préférée entre 1/4 et 1/10 de l'épaisseur de l'équipement industriel à évaluer,
   - ledit capteur étant réalisé directement dans une partie de paroi de l'équipement industriel à évaluer.

2. Méthode d'évaluation du risque de fragilisation par l'hydrogène d'un métal dans un environnement donné, utilisant l'équipement industriel selon la revendication 1, dans laquelle on procède en suivant les étapes suivantes :

   - Etape 1 : on procède pour un métal donné à des tests de fissuration dans des environnements hydrogénant présentant des risques de fragilisation par l'hydrogène croissants, afin d'obtenir d'une part les conditions pour lesquelles il n'y a pas de fissuration et d'autre part les conditions pour lesquelles une fissuration est rencontrée,
   - Etape 2 : on procède, dans les mêmes environnements hydrogénant que pour l'étape 1, à des tests de mesure de pression à l'aide du capteur, réalisé dans le même métal que celui utilisé pour l'étape 1, et on mesure la pression d'équilibre (Pe) en H2 pour chaque environnement,
   - Etape 3 : on compare pour chaque environnement les résultats des tests de fissuration et des tests de mesure de pression, et on détermine quelle est la pression minimale correspondant à des situations de fissuration. Cette pression minimale est considérée comme la valeur seuil de résistance à la fissuration pour cet acier, et est désignée Ps,

   - Etape 4 : on expose le capteur réalisé dans le même métal à un environnement donné, et on suit l'évolution dans le temps de la pression dans le capteur jusqu'à ce que celle-ci atteigne un plateau où elle devient constante. On désigne cette pression d'équilibre comme Pe,
   - Etape 5 : on compare alors la valeur à l'équilibre Pe avec la valeur seuil Ps obtenue à l'étape 3 :

      * Si Pe est inférieure à Ps, il n'y a pas de risque de fissuration de l'acier dans les conditions considérées,
      * si Pe est supérieure à Ps, il y a un risque de fissuration.

3. Méthode selon la revendication 2, pour évaluer le risque de fragilisation d'aciers soumis à des environnements contenant de l'H2 ou de l'H2S et présentant des pH de 3 à 8, et préférentiellement de 4 à 7.

## Patentansprüche

1. Industrielle Betriebseinrichtung, wobei es sich bei dieser industriellen Betriebseinrichtung um einen Reaktor oder eine Rohrleitung handelt, umfassend einen Sensor zum Messen der Gefahr einer Wasserstoffversprödung des Metalls der zu untersuchenden industriellen Betriebseinrichtung, umfassend die folgenden Elemente:

   - einen Metallkörper mit einem darin hergestellten abgeschlossenen Hohlraum, wobei dieser Hohlraum an einem seiner Enden eine Kommunikation mit einer Druckmessvorrichtung aufweist,
   - eine Druckmessvorrichtung, die mit dem Hohlraum verbunden ist und mit der sich der Druck im Inneren des Hohlraums messen lässt,
   - wobei der Hohlraum eine zwischen der äußeren und der inneren Oberfläche des Hohlraums gemessene Wandstärke zwischen 1/3 und 1/50 und vorzugsweise zwischen 1/4 und 1/10 der Dicke der zu untersuchenden industriellen Betriebseinrichtung hat,
   - wobei der Sensor direkt in einem Teil der Wand der zu untersuchenden industriellen Betriebseinrichtung realisiert ist.

2. Verfahren zur Untersuchung der Gefahr einer Wasserstoffversprödung eines Metalls in einer gegebenen Umgebung unter Verwendung der industriellen Betriebseinrichtung nach Anspruch 1, bei dem man die folgenden Schritte durchführt:

   - Schritt 1: man führt an einem gegebenen Metall Rissprüfungen in hydrierenden Umgebungen durch, in denen eine zunehmende Gefahr einer Wasserstoffversprödung besteht, um einerseits Bedingungen, bei denen keine Rissbildung vorliegt, und andererseits Bedingungen, bei denen Rissbildung beobachtet wird, zu erhalten,

- Schritt 2: in den gleichen hydrierenden Umgebungen wie in Schritt 1 werden Druckmessungstests mithilfe eines Sensors, der in dem gleichen Metall, wie es für Schritt 1 verwendet wird, realisiert ist, durchgeführt und man misst den $H_2$-Gleichgewichtsdruck (Pe) für jede Umgebung;

- Schritt 3: man vergleicht für jede hydrierende Umgebung der Ergebnisse der Rissprüfungen und der Druckmesstests und man bestimmt, welches der Mindestdruck ist, der Rissbildungssituationen entspricht. Dieser Mindestdruck wird als Schwellenwert für die Rissbeständigkeit bei diesem Stahl angesehen und als Ps bezeichnet,

- Schritt 4: man setzt den in demselben Metall realisierten Sensor einer gegebenen Umgebung aus und verfolgt die Veränderung des Drucks in dem Sensor mit der Zeit bis der Druck ein Plateau erreicht oder konstant wird. Dieser Gleichgewichtsdruck wird als Pe bezeichnet,

- Schritt 5: dann vergleicht man den Gleichgewichtswert Pe mit dem im Schritt 3 erhaltenen Schwellenwert Ps:

* Wenn Pe kleiner ist als Ps, besteht keine Gefahr einer Wasserstoffversprödung des Stahls unter den betrachteten Bedingungen,
* wenn Pe größer ist als Ps, besteht die Gefahr einer Wasserstoffversprödung.

3. Verfahren nach Anspruch 2 zur Untersuchung der Gefahr einer Wasserstoffversprödung von Stählen, die $H_2$ oder $H_2S$ enthaltenden Umgebungen mit pH-Werten von 3 bis 8 und vorzugsweise von 4 bis 7 ausgesetzt sind.

## Claims

1. Industrial equipment, said industrial equipment being a reactor or a pipeline, comprising a sensor for the measurement of the risk of embrittlement by hydrogen of the metal of the industrial equipment to be evaluated, comprising the following elements:

- a metal body, in which a closed cavity is produced, this cavity exhibiting, at one of its ends, a communication with a pressure measurement device,
- a device for measurement of the pressure connected to the cavity and which makes it possible to measure the pressure inside said cavity,
- said cavity having a wall thickness, measured between the exterior surface and interior surface of the cavity, of between 1/3 and 1/50 and preferably between 1/4 and 1/10 of the thickness of the industrial equipment to be evaluated,
- said sensor being produced directly in a wall part of the industrial equipment to be evaluated.

2. Method for evaluation of the risk of embrittlement by hydrogen of a metal in a given environment, using the industrial equipment according to Claim 1, in which the procedure is carried out by following the following stages:

- Stage 1: for a given metal, cracking tests are carried out in hydrogenating environments exhibiting increasing risks of embrittlement by hydrogen, in order to obtain, on the one hand, the conditions for which there is no cracking and, on the other hand, the conditions for which cracking is encountered,
- Stage 2: pressure measurement tests are carried out, in the same hydrogenating environments as for Stage 1, using the sensor, made from the same metal as that used for Stage 1, and the $H_2$ equilibrium pressure (Pe) is measured for each environment,
- Stage 3: the results of the cracking tests and of the pressure measurement tests are compared for each environment and it is determined what is the minimum pressure corresponding to cracking situations; this minimum pressure is regarded as the threshold cracking resistance value for this steel, and is denoted Ps,
- Stage 4: the sensor made from the same metal is exposed to a given environment and the change over time of the pressure in the sensor is monitored until the pressure reaches a plateau, where it becomes constant; this equilibrium pressure is denoted as Pe,
- Stage 5: the value at equilibrium Pe is then compared with the threshold value Ps obtained in Stage 3:

* if Pe is less than Ps, there is no risk of cracking of the steel under the conditions under consideration,
* if Pe is greater than Ps, there is a risk of cracking.

3. Method according to Claim 2, for evaluating the risk of embrittlement of steel subjected to environments containing $H_2$ or $H_2S$ and exhibiting pH values from 3 to 8 and preferentially from 4 to 7.

(3)

(2)

(1)

P

**FIGURE 1**

(3)

(1)

(2)

P

**FIGURE 1 BIS**

Figure 2

Figure 3

Figure 4

Figure 5

a) Test de fissuration
Mesure de l'étendue de la fissuration pour différentes conditions (différentes solutions d'essai).

b) Test de mesure de pression
Mesure de la pression d'équilibre pour différentes conditions (différentes solutions d'essai).

Figure 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2011179893 A
- JP 2013044712 A
- JP 2013044716 A
- US 5279169 A
- US 6537824 B
- US 4416996 A
- WO 13012364 A1
- US 6058765 A
- US 2013236975 A